# EUROPEAN PATENT SPECIFICATION

(11) **EP 4 382 281 B1**
(45) Date of publication and mention of the grant of the patent: **29.10.2025**
(21) Application number: 22212802.7
(22) Date of filing: 12.12.2022
(51) Int. Cl.: B29C 64/106, B29C 64/118, B29C 64/209, B29C 64/227, B29C 64/25, B29C 64/336, B29C 64/364, B29C 64/393, B33Y 30/00, B33Y 50/02, B33Y 70/10, B29C 64/379, B33Y 40/20

(54) **INTEGRATED BIOMANUFACTURING SYSTEM FOR PRODUCING THREE-DIMENSIONAL STRUCTURES**
INTEGRIERTES BIOHERSTELLUNGSSYSTEM ZUR HERSTELLUNG DREIDIMENSIONALER STRUKTUREN
SYSTÈME DE BIOFABRICATION INTÉGRÉ POUR PRODUIRE DES STRUCTURES TRIDIMENSIONNELLES

(30) Priority: 07.12.2022 PT 2022118381
(43) Date of publication of application: 12.06.2024
(73) Proprietor: Instituto Politécnico De Leiria, 2411-901 Leiria (PT)
(72) Inventor: FERNANDES ALVES, NUNO MANUEL, 2430-028 Marinha Grande (PT); RODRIGUES PASCOAL FARIA, PAULA CRISTINA, 2430-028 Marinha Grande (PT); ABREU VALENTE, JOANA FILIPA, 2430-028 Marinha Grande (PT); ROSA DIAS, JULIANA, 2430-028 Marinha Grande (PT); FERNANDES PATRICIO, TATIANA MARISA, 2430-028 Marinha Grande (PT); DA SILVA BISCAIA, SARA ISABEL, 2430-028 Marinha Grande (PT); DA SILVA CARREIRA, PEDRO JOSÉ, 2430-028 Marinha Grande (PT)
(74) Representative: Monteiro Alves, Inês

(56) References cited:
- CN-A- 106 827 496
- US-A1- 2016 068 793
- US-A1- 2019 232 558
- US-A1- 2020 384 690
- US-A1- 2021 395 675

## Description

### TECHNICAL FIELD

This invention refers to an integrated biomanufacturing system for producing three-dimensional structures, especially those developed for treating degenerative diseases involving the regeneration of tissue, such as bone and cartilage, simultaneously.

### PRIOR ART

Degenerative diseases, such as osteoarthritis (OA), for example, have a significant impact on the world in economic and social terms, this being the most prevalent degenerative articular disease in human beings and in other animal species (for example, horses, dogs, etc.).

Various devices and drugs currently available help remedy diseases associated to the degeneration of tissue(s) (bone and cartilage). However, they oftentimes cause adverse effects that may prolong the treatment or, furthermore, be ineffective, leading to an increase in costs for the health system in question and to a lowering of the quality of life of people and other animal species.

This situation can be minimized by using three-dimensional structures with biomimetic properties, which promote the regeneration process of the native tissue(s).

In light of the context referred to above, studies have been carried out with the aim of devising alternative technologies that could be useful in the treatment of diseases associated to the degeneration of native tissue(s).

Accordingly, various models of three-dimensional structures (3D) or scaffolds are already known in the state of the art, having, however, important limitations in the inability: (i) to replicate the biomechanical environment of the native tissue or multi-tissue; (ii) to prolong and / or control the release *"in situ"* of the pharmacological models; and (iii) to provide multiple signaling biomolecules and to simultaneously regenerate avascular tissue (cartilage) and vascular tissue (bone), signifying a deficient articulation regeneration.

Though deemed an important strategy, the clinical application of the existing techniques is still quite limited due to the high mortality of the cells caused by the inflammatory process *"in situ"* and to the inability to control the process of functional regeneration.

Currently, the biomanufacturing systems for obtaining existing 3D structures include:
- A hybrid system that uses electrospinning techniques and jet printing to produce structures with improved mechanical and biological properties for the regeneration of cartilage tissue. In this system, the three-dimensional structures are obtained by successively adding layers alternating poly (ε-caprolactone) (PCL) with collagen hydrogel - fibrin incorporating cells (rabbit chondrocytes) (Atala A. *et al,* 2012);
- A portable biomanufacturing tool (more specifically, a pen loaded with biomaterial named *"Biopen*") that enables the 3D printing and manual control during the deposition of the three-dimensional structure during the course of the surgical procedure with or without live cells (O'Connell C. *et al,* 2016). The *"Biopen"* system basically consists of an extrusion-based printer which was tested in the development of "core/shell GelMa/HAMa" three-dimensional structures that have a mechanical strength of 200 KPa and good cellular viability for repairing the chondral tissue (Duchi S. *et al,* 2017). The "Biopen" system was also used to study the reparation of defects of the chondral tissue with total thickness in an ovine model, presenting clinical potential for three-dimensional bioprinting *"in situ"* (Di Bella C. *et al,* 2018);
- A fast prototyping system named *"3D Bioplotter",* mainly used to process various biomaterials for computer-assisted tissue engineering, from 3D digital models and computerized tomography data of patients up to the production of 3D structures (Webpage [1]). This system enables three-dimensional structures to be produced, also in sterile environments, using a broad variety of materials such as hydrogels, thermoplastic polymers, ceramics and metals. This system is not limited to the use of materials from the supplier, and the user can choose his or her own materials in accordance with the specific needs in medical applications.
- The company CELLINK developed various 3D bioprinting systems, among the most advanced existing on the market, named BIO-CELLX (Webpage [2]). The BIO-CELLX system enables the preparation of culture cells, the selection of biomaterials and automatic mixing thereof with cells. Further, the system also enables cross-linking of biomaterials through a photo-cross-linking module which is built in with thermal extrusion modules of bio-thermoplastics. This system also includes a new mechanism for distributing biomaterials / cells (biodispensing, which is claimed in patent application EP3862086A1), enabling the creation of physiologically advanced three-dimensional structures. These structures are obtained in a fully closed environment, guaranteeing the sterility of all the work flow from the preparation of the biomaterials / cells up to the final production of the structure, which may also present uniformly distributed cellular density, guaranteeing reproducible 3D models in the mixing wells for cellular culture.

US 2016/068793 A1 discloses an integrated biomanufacturing system for producing three-dimensional structures, comprising modules that can operate in synchronism, wherein said modules are a bioextrusion module comprising at least one extrusion head, at least one deposition head and a sliding work platform, an automatic monitoring module in situ comprising a digital chamber, a light source and a processing unit and a culture module in vitro comprising a bioreactor, wherein the bioextrusion module is configured to enable the extrusion of filaments with diameters on a micro or macro scale, wherein said integrated biomanufacturing system is completely involved by a laminar flow chamber.

Differently to the biomanufacturing systems mentioned above, the present invention describes a new integrated biomanufacturing system, which enables the production of new three-dimensional biomimetic structures which present morphological, physical, thermal, mechanical, chemical and biological properties which mimic the native tissue(s) (such as bone and / or cartilage), being of crucial importance for medical applications involving multi-tissue problems.

The present integrated biomanufacturing system enables new three-dimensional mono or multi-tissue structures (scaffolds) to be manufactured which mimic the biomechanical environment of the native tissue(s), such as three-dimensional multi-tissue hierarchical structures that may have up to six different regions which mimic the extracellular matrix (ECM) associated to the bone (subchondral and trabecular zones) and to the cartilage (superficial, middle, deep and calcified cartilage zones).

Accordingly, the integrated biomanufacturing system of the present invention comprises four modules: (i) a bioextrusion module, which combines the co-extrusion of biomaterials with the deposition of hydrogels (with or without drugs and encapsulated cells); (ii) an automatic monitoring module *in situ* enabling control and digital reconstruction 3D of scaffolds; (iii) an electrospinning module that enables the production of fiber meshes, random or aligned with diameters on a nano / micrometer scale; and (iv) a culture module *in vitro* in a bioreactor that enables cellular culture to be carried out in a fully integrated manner.

Said integrated biomanufacturing system is useful for producing three-dimensional structures, especially those developed for treating degenerative diseases involving the regeneration of tissue, such as bone and cartilage, simultaneously.

The invention enables the production of three-dimensional structures whose monitoring / digital reconstruction (on a nano to macro scale) is performed *"in situ",* automatically, in synchronism and in real time.

Further, the culture module *"in vitro"* provides the ideal biomechanical environment for producing three-dimensional structures and the implementation of cellular culture in an integrated fashion.

### PROBLEMS WITH PRIOR ART

Most of the studies and publications relating to biomanufacturing systems enables three-dimensional structures or scaffolds to be obtained with some ability for mimicking the biomechanical environment of the native tissue.

However, these systems produce three-dimensional structures that:
- do not replicate the various layers of the mono-tissue (for example, 2 layers of bone or 4 layers of cartilage);
- do not have any ability to mimic various native tissues simultaneously (for example, bone and cartilage);
- do not have the ability to carry out, in an integrated manner, the dynamic cellular culture by way of a suitable bioreactor, whereby compromising the native biomechanical environment required by cellular culture.

Although the steps forward referred to previously in the development of biomanufacturing systems of three-dimensional structures, it is not possible to perform the monitoring / supervision / 3D digital reconstruction *"in situ"* and in real time of same.

The architecture on different scales (micro to macro) of the three-dimensional structures is typically reconstructed (recovered) digitally, once production thereof has been finalized, using very expensive and lengthy techniques that are not integrated into the biomanufacturing systems, such as, for example, computerized x-ray microtomography (MicroCT) and scanning electron microscopy (SEM).

### ABSTRACT OF THE INVENTION

This invention describes an integrated biomanufacturing system for producing three-dimensional structures comprising four modules that can operate individually, or in partial or total synchronism, namely:
- a bioextrusion module comprising at least one extrusion head, at least one deposition head and a sliding work platform;
- an automatic monitoring module *in situ* comprising a digital chamber, a light source and a processing unit;
- an electrospinning module comprising a flat and/or rotary collector, a syringe with needle/nozzle and a peristaltic pump; and
- a culture module *in vitro* comprising a bioreactor;

wherein the bioextrusion module is configured to enable the extrusion of filaments with diameters on a micro and / or macro scale;
wherein the automatic monitoring module *in situ* is configured to enable the digital reconstruction of the three-dimensional structure by reconfiguring the manufacturing trajectories;
wherein the electrospinning module is configured to produce simple and / or tubular nanofibers, oriented in a random or aligned manner in accordance with the preferred direction;
wherein the culture module *in vitro* is configured to enable the differentiation and / or the proliferation of the cells encapsulated in the three-dimensional structure in a dynamic way; and
wherein said integrated biomanufacturing system is completely involved by a laminar flow chamber.

### SOLUTION TO THE PROBLEM

The present invention solves countless problems of the state of the art related to the three-dimensional structures utilized in the treatment of degenerative diseases involving the regeneration of tissue simultaneously, such as bone and cartilage, this tissue being a mono tissue or a multi-tissue.

A first point concerns the inflammation and rejection processes deriving from the use of mono-tissue substitutes, which, due to their characteristics, do not take into consideration the specificities of the patient.

A second point refers to the absence, at a laboratory / commercial level, of multi-tissue substitutes that consider the treatment of degenerative diseases involving more than one native tissue, such as the articulations (which comprise bone and cartilage). Accordingly, what is needed are three-dimensional structures that replicate the various layers of the tissues to be regenerated.

In the present invention, the integrated biomanufacturing system enables three-dimensional biomimetic structures to be obtained which mimic the native tissues in terms of organization, morphology and mechanical and biological properties.

Furthermore, the integrated biomanufacturing system is comprised of four modules, which can operate individually, or in partial or total synchronism, for producing hierarchical three-dimensional structures and dynamic cellular culture *"in situ",* in a totally sterilized environment, whereby enabling a closed work cycle from selecting the biomaterials, the bioactive cells and molecules, up to and including the dynamic cellular culture.

Due to its innumerous advantages, the invention contributes as an effective and innovative alternative for producing three-dimensional biological substitutes for treating degenerative problems involving more than one native tissue.

### ADVANTAGEOUS EFFECTS OF THE INVENTION

Among the main advantageous effects of the present invention, the presence of four modules with different functions are of particular significance: (i) bioextrusion, (ii) automatic monitoring *in situ,* (iii) electrospinning and (iv) culture *in vitro,* wherein said modules can operate individually, or in partial or total synchronism.

The integration between the modules enables the incorporated production of three-dimensional multi-tissue hierarchical structures, that is, three-dimensional biological substitutes that have up to six different regions which mimic the extracellular matrix of bones and cartilages.

Additionally, the integration between the modules also enables the association of the three-dimensional structures to the dynamic cellular culture *"in situ",* promoting the native biomechanical environment required by the culture.

### BRIEF DESCRIPTION OF THE DRAWINGS

With the aim of promoting an understanding of the principles in accordance with the embodiments of the present invention, reference will now be made to the embodiments illustrated in the drawings and to the language used to described them.

It should be understood that there is no intention of limiting the ambit of the invention to the content of the drawings and that modifications of the inventive characteristics illustrated, as well as additional applications of the principles and embodiments illustrated, which would occur normally for a person skilled in the art in possession of this specification, are considered within the ambit of the invention now claimed.

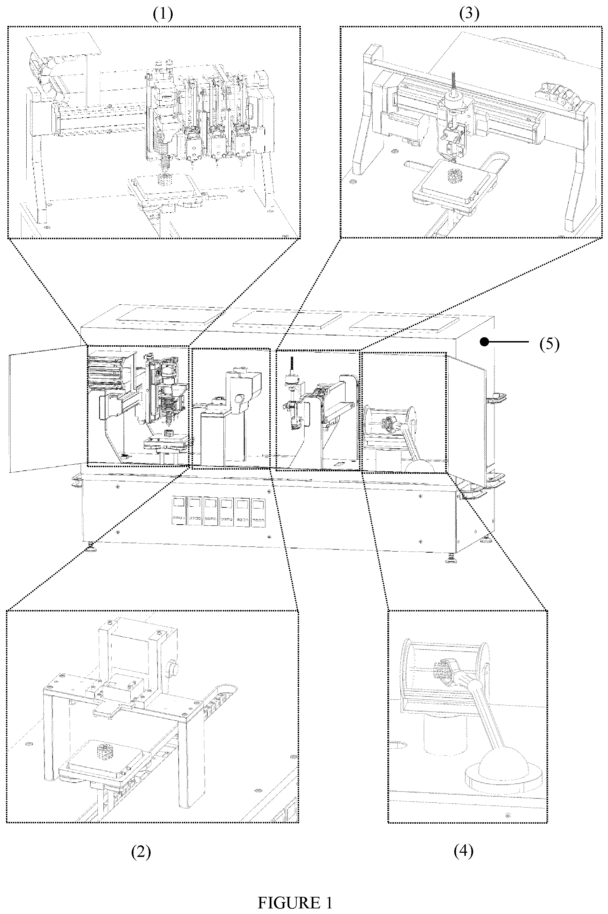

Figure 1 - illustrates the integrated biomanufacturing system of the present invention, which comprises four modules, namely: (1) a bioextrusion module, (2) an automatic monitoring module *in situ,* (3) an electrospinning module, and (4) a culture module *in vitro* with bioreactor, wherein the system is completely involved by a laminar flow chamber(5).

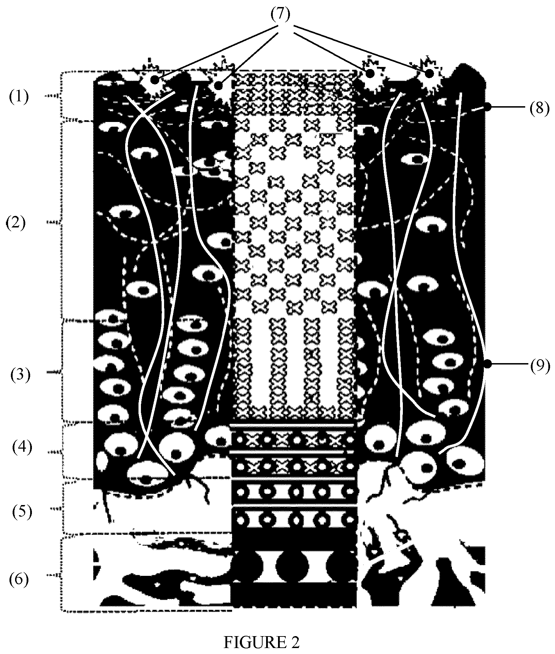

Figure 2 - illustrates a cross-section of the six layers of the articular region, namely: (1) the superficial tangential zone of the cartilage (thickness of 10% to 20% of the total thickness), (2) the middle transition zone of the cartilage (thickness of 40% to 60% of the total thickness), (3) the deep radial zone of the cartilage (thickness of 30% to 40% of the total thickness), (4) the calcified cartilage zone, (5) the subchondral bone zone, (6) the trabecular bone zone, (7) development of superficial degeneration of the cartilage, (8) rupture of collagen fibers in degenerative process, and (9) long, healthy collagen fibers.

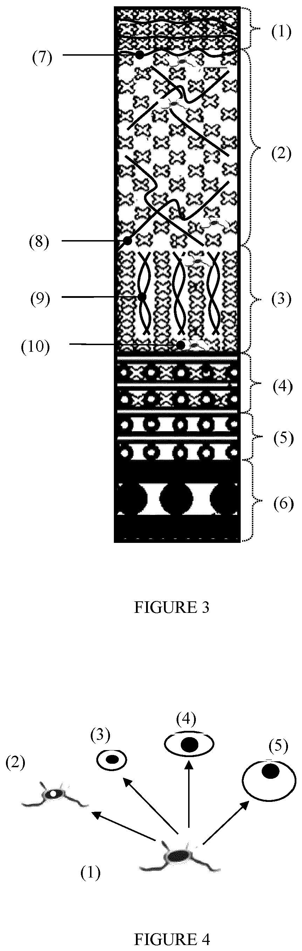

Figure 3 - illustrates a multi-tissue hierarchical three-dimensional structure manufactured by the integrated biomanufacturing system of the present invention comprising: (1) (1) biomechanical mimicking of the superficial tangential zone of the cartilage, (2) biomechanical mimicking of the middle transition zone of the cartilage, (3) biomechanical mimicking of the deep radial zone of the cartilage, (4) biomechanical mimicking of the calcified cartilage zone, (5) biomechanical mimicking of the subchondral bone zone, (6) biomechanical mimicking of the trabecular bone zone, (7) collagen fibers horizontally aligned, (8) collagen fibers distributed randomly, (9) long collagen fibers aligned vertically, and (10) Mesenchymal stem cells - non-differentiated encapsulated in the 3D structure.
Figure 4 - illustrates the cell differentiation mechanism, comprising: (1) mesenchymal stem cells - non-differentiated and encapsulated in the 3D structure, (2) neurons, (3) osteocytes (bone cells), (4) chondrocytes (cartilage cells), and (5) adipocytes (fatty cells).

### DESCRIPTION OF THE EMBODIMENTS

The present invention refers to an integrated biomanufacturing system for producing three-dimensional structures, which is represented in Figure 1.

Said integrated biomanufacturing system comprises four modules which can operate individually, or in partial or total synchronism, namely:
- a bioextrusion module comprising at least one extrusion head, at least one deposition head and a sliding work platform;
- an automatic monitoring module *in situ* comprising a digital chamber, a light source and a processing unit;
- an electrospinning module comprising a flat and / or rotary collector, a syringe with needle / nozzle and a peristaltic pump; and
- a culture module *in vitro* comprising a bioreactor;
wherein said integrated biomanufacturing system is completely involved by a laminar flow chamber.

In a preferred embodiment of the present invention, the bioextrusion module enables the extrusion of filaments with diameters on a micro and / or macro scale and comprises at least one extrusion head of at least one polymer material and at least one deposition head of various biomaterials. The at least one extrusion head and the at least one deposition head move horizontally and vertically according to the axes y and z.

Said at least one extrusion head consists of a liquefaction device made of at least one bio-thermoplastic polymer coupled to a thread and nozzle head to create the cast polymer flow and subsequent deposition on the sliding work platform.

Said at least one deposition head deposits natural biomaterials, biomolecules / growth factors and cells (differentiated / non-differentiated), thus enabling the production of three-dimensional hybrid structures and / or with cells encapsulated.

In a preferred embodiment of the present invention, the bio-thermoplastic polymer may be a polymer selected from the group consisting of poly (ε-caprolactone), poly lactic acid, poly lactic acid and glycolic among others, as well as combinations thereof.

In a preferred embodiment of the present invention, the natural biomaterials are selected from the group consisting of hydrogels, gelatin, collagen, recombinant peptides, among others, as well as combinations thereof.

In a preferred embodiment of the present invention, the biomolecules / growth factors are β3 (TGFβ3), among others.

In a preferred embodiment of the present invention, the cells are selected from the group consisting of mesenchymal cells - non-differentiated.

In a preferred embodiment of the present invention, the bioextrusion module further comprises a sliding work platform, configured to move along the x axis.

This bioextrusion technique is highly reproducible and enables three-dimensional structures to be obtained with superior mechanical properties, controlled porosity (size, shape and distribution of pores) and lower decomposition rates.

In a preferred embodiment of the present invention, the automatic monitoring module *in situ* enables a digital reconstruction of the three-dimensional structure. Accordingly, the digital chamber, with the help of the light source, captures a digital image of the three-dimensional structure and accurately detects relevant data thereof on a greyscale, such as the diameter and the length of the filaments, the diameter and the length of the electrospinning fibers, the geometry of the pores and the total porosity of the three-dimensional structure.

Next, a processing unit performs, by way of software, the analysis of the data obtained and enables the digital reconstruction of the three-dimensional structure by reconfiguring the manufacturing trajectories, for example, of the deposition speed and of the rotation speed of the extrusion screw.

In a preferred embodiment of the present invention, the electrospinning module is configured for producing simple and / or tubular nanofibers, oriented randomly or aligned in accordance with a preferred direction, with the ability to incorporate cells and / or drugs.

In a preferred embodiment of the present invention, the syringe and needle / nozzle of the electrospinning module move horizontally and vertically, according to the y and z axes, and the sliding work platform moves on the x axis.

In a preferred embodiment of the present invention, the culture module *in vitro* comprises a bioreactor which makes travel and / or rotation movements and is configured for the dynamic cultivation of the cells encapsulated in the three-dimensional structure, whereby allowing the differentiation and / or the proliferation of the cells of interest (as shown in Figure 4).

In a preferred embodiment of the present invention, the specific cellular lines are selected in accordance with the intended clinical application, based on mimicking the natural biomechanical environment of the tissue(s).

The laminar flow chamber that involves all the integrated biomanufacturing system of the present invention guarantees the suitable sterility conditions for producing 3D structures for biomedical applications. In particular, the present integrated biomanufacturing system enables hierarchical mono or multi-tissue three-dimensional structures (scaffolds) to be manufactured which mimic the biomechanical environment of the native tissue(s).

In a preferred embodiment of the present invention, the three-dimensional multi-tissue hierarchical structures have up to six different regions which mimic the extracellular bone matrix (subchondral and trabecular zones) and cartilages (superficial, middle, deep and calcified cartilage zones).

Figures 2 and 3 are schematic representations showing the cut view of the six layers of the osteochondral region of an articulation and a three-dimensional hierarchical multi-tissue structure manufactured by the integrated biomanufacturing system of the present invention, respectively.

The subject matter-object described above is provided as an illustration of the present invention and, therefore, should not be interpreted in a way that limits it. The terminology employed with the purpose of describing preferred embodiments of the present invention should not restrict it to same.

As used in the specification, the definite and indefinite articles, in their singular form, are also meant to include their plural forms, unless the context of the description explicitly indicates the opposite.

The indefinite article "a" or "an" should be interpreted generally as "one or more", unless the sense of a singular modality is clearly defined in a specific situation.

It shall be understood that the terms "comprise" and "include", when used in this specification, define the present of characteristics, elements, components, steps and related operations, but do not exclude the possibility of other characteristics, elements, components, steps and operations also being encompassed.

As used throughout this patent application, the term "or" is employed in the sense of being inclusive as opposed to exclusive, unless the exclusive sense is clearly defined in a specific situation. In this context, a phrase of the type "X uses A or B" should be interpreted as including all the pertinent inclusive combinations, for example "X uses A", "X uses B" and "X uses A and B".

All the alterations, provided that they do not modify the essential characteristics of the accompanying claims, should be considered as within the ambit of protection of the present invention.

### LIST OF CITATIONS

Below is the list of citations:

### PATENT LITERATURE

- EP3862086A1

### NON-PATENT LITERATURE

Atala A, Yoo J, Zhao W, Dice D, Binder K, Xu T, Albanna M (2012). Hybrid printing of mechanically and biologically improved constructs for cartilage tissue engineering applications. Biofabrication, 5, 015001.

O'Connell C, Di Bella C, Thompson F, Augustine C, Beirne S, Cornock R, Richards C, Chung J, Gambhir S, Yue Z, Bourke J, Zhang B, Taylor A, Quigley A, Kapsa R, Choong P, Wallace G (2016). Development of the biopen: a handheld device for surgical printing of adipose stem cells at a chondral wound site. Biofabrication, 8, 015019.

Duchi S, Onofrillo C, O'Connell C, Blanchard R, Augustine C, Quigley A, Kapsa R, Pivonka P, Wallace G, Di Bella C, Choong P (2017). Handheld coaxial bioprinting: application to in situ surgical cartilage repair, Sci. Rep., 7, 1 - 12.

Di Bella C, Duchi S, O'Connell C, Blanchard R, Augustine C, Yue Z, Thompson F, Richards C, Beirne S, Onofrillo C, Bauquier S, Ryan S (2018). In situ handheld three-dimensional bioprinting for cartilage regeneration. J. Tissue Eng. Regenerat. Med., 12, 611 - 621.
Webpage [1]: https://3dsman.com/envisiontec-3d-bioplotter/
   (accessed on November 28, 2022)
Webpage [2]: https://www.cellink.com/bio-cellx/
   (accessed on November 28, 2022)

## Claims

1. An integrated biomanufacturing system for producing three-dimensional structures **characterized by** comprising four modules that can operate individually or in partial or total synchronism, wherein said modules are:
- a bioextrusion module (1) comprising at least one extrusion head, at least one deposition head and a sliding work platform;
- an automatic monitoring module *in situ* (2) comprising a digital chamber, a light source and a processing unit;
- an electrospinning module (3) comprising a flat and / or rotary collector, a syringe with needle / nozzle and a peristaltic pump; and
- a culture module *in vitro* (4) comprising a bioreactor;
wherein the bioextrusion module is configured to enable the extrusion of filaments with diameters on a micro and / or macro scale;
wherein the automatic monitoring module *in situ* is configured to enable the digital reconstruction of the three-dimensional structure by reconfiguring the manufacturing trajectories;
wherein the electrospinning module is configured to produce simple and / or tubular nanofibers, oriented randomly or aligned in accordance with a preferred direction;
wherein the culture module *in vitro* is configured to enable the differentiation and / or the proliferation of the cells encapsulated in the three-dimensional structure in a dynamic way; and
wherein said integrated biomanufacturing system is completely involved by a laminar flow chamber (5).

2. The integrated biomanufacturing system according to claim 1, **characterized in that** the at least one extrusion head consists of a liquefaction device of at least one bio-thermoplastic polymer coupled to a thread and nozzle head.

3. The integrated biomanufacturing system according to claim 1, **characterized in that** the at least one deposition head deposits natural biomaterials, biomolecules / growth factors and differentiated and / or non-differentiated cells.

4. The integrated biomanufacturing system according to claim 1, **characterized in that** the at least one extrusion head and the at least one deposition head move horizontally and vertically according to the y and z axes, and the sliding work platform moves on the x axis.

5. The integrated biomanufacturing system according to claim 1, **characterized in that** the digital chamber captures a digital image of the three-dimensional structure and accurately detects relevant data thereof, which will be handled by the processing unit.

6. The integrated biomanufacturing system according to claim 1, **characterized in that** the processing unit carries out the analysis of data obtained by means of softwares.

7. The integrated biomanufacturing system according to claim 1, **characterized in that** the syringe and needle / nozzle move horizontally and vertically according to the y and z axes, and the sliding work platform moves on x axis.

8. The integrated biomanufacturing system according to claim 1, **characterized in that** the bioreactor performs travel and / or rotation movements.

## Patentansprüche

1. Ein integriertes Bioherstellungssystem zur Herstellung dreidimensionaler Strukturen, **dadurch gekennzeichnet, dass** es vier Module umfasst, die einzeln oder teilweise oder vollständig synchron betrieben werden können, wobei die Module folgende sind:
- ein Bioextrusionsmodul (1), das mindestens einen Extrusionskopf, mindestens einen Abscheidungskopf und eine verschiebbare Arbeitsplattform umfasst;
- ein automatisches Überwachungsmodul in situ (2), das eine digitale Kammer, eine Lichtquelle und eine Verarbeitungseinheit umfasst;
- ein Elektrospinnmodul (3), das einen flachen und/oder rotierenden Kollektor, eine Spritze mit Nadel/Düse und eine Peristaltikpumpe umfasst; und
- ein In-vitro-Kulturmodul (4), das einen Bioreaktor umfasst;
wobei das Bioextrusionsmodul so konfiguriert ist, dass es die Extrusion von Filamenten mit Durchmessern im Mikro- und/oder Makrobereich ermöglicht;
wobei das automatische Überwachungsmodul in situ so konfiguriert ist, dass es die digitale Rekonstruktion der dreidimensionalen Struktur durch Neukonfiguration der Fertigungsbahnen ermöglicht;
wobei das Elektrospinnmodul so konfiguriert ist, dass es einfache und/oder röhrenförmige Nanofasern erzeugt, die zufällig ausgerichtet oder gemäß einer bevorzugten Richtung ausgerichtet sind;
wobei das In-vitro-Kulturmodul so konfiguriert ist, dass es die Differenzierung und/oder Proliferation, der in der dreidimensionalen Struktur eingekapselten Zellen auf dynamische Weise ermöglicht; und
wobei das integrierte Bioherstellungssystem vollständig von einer Laminarströmungskammer (5) umgeben ist.

2. Das integrierte Bioherstellungssystem nach Anspruch 1, **dadurch gekennzeichnet, dass** der mindestens eine Extrusionskopf aus einer Verflüssigungsvorrichtung für mindestens ein biothermoplastisches Polymer besteht, die mit einem Faden und einem Düsenkopf verbunden ist.

3. Das integrierte Bioherstellungssystem nach Anspruch 1, **dadurch gekennzeichnet, dass** der mindestens eine Abscheidungskopf natürliche Biomaterialien, Biomoleküle/Wachstumsfaktoren und differenzierte und/oder nicht differenzierte Zellen abscheidet.

4. Das integrierte Bioherstellungssystem nach Anspruch 1, **dadurch gekennzeichnet, dass** der mindestens eine Extrusionskopf und der mindestens eine Abscheidungskopf sich horizontal und vertikal entlang der y- und z-Achse bewegen und die verschiebbare Arbeitsplattform sich auf der x-Achse bewegt.

5. Das integrierte Bioherstellungssystem nach Anspruch 1, **dadurch gekennzeichnet, dass** die digitale Kammer ein digitales Bild der dreidimensionalen Struktur aufnimmt und relevante Daten davon genau erfasst, die von der Verarbeitungseinheit verarbeitet werden.

6. Das integrierte Bioherstellungssystem nach Anspruch 1, **dadurch gekennzeichnet, dass** die Verarbeitungseinheit die Analyse der gewonnenen Daten mittels Software durchführt.

7. Das integrierte Bioherstellungssystem nach Anspruch 1, **dadurch gekennzeichnet, dass** die Spritze und die Nadel/Düse sich horizontal und vertikal entlang der y- und z-Achse bewegen und die verschiebbare Arbeitsplattform sich auf der x-Achse bewegt.

8. Das integrierte Bioherstellungssystem nach Anspruch 1, **dadurch gekennzeichnet, dass** der Bioreaktor Bewegungs- und/oder Drehbewegungen ausführt.

## Revendications

1. Le système de biofabrication intégré pour produire des structures tridimensionnelles, **caractérisé en ce qu'**il comprend quatre modules pouvant fonctionner individuellement ou en synchronisme partiel ou total, dans lequel lesdits modules sont :
- un module de bioextrusion (1) comprenant au moins une tête d'extrusion, au moins une tête de dépôt et une plate-forme de travail coulissante ;
- un module de surveillance automatique in situ (2) comprenant une chambre numérique, une source lumineuse et une unité de traitement
- un module d'électrofilage (3) comprenant un collecteur plat et/ou rotatif, une seringue avec aiguille/buse et une pompe péristaltique ; et
- un module de culture in vitro (4) comprenant un bioréacteur ;
dans lequel le module de bioextrusion est configuré pour permettre l'extrusion de filaments avec des diamètres à l'échelle micro et/ou macro ;
dans lequel le module de surveillance automatique in situ est configuré pour permettre la reconstruction numérique de la structure tridimensionnelle en reconfigurant les trajectoires de fabrication ;
dans lequel le module d'électrofilage est configuré pour produire des nanofibres simples et/ou tubulaires, orientées de manière aléatoire ou alignées selon une direction préférée ;
dans lequel le module de culture in vitro est configuré pour permettre la différenciation et/ou la prolifération des cellules encapsulées dans la structure tridimensionnelle de manière dynamique ; et
dans lequel ledit système de biofabrication intégré est entièrement entouré par une chambre à écoulement laminaire (5).

2. Le système de biofabrication intégré selon la revendication 1, **caractérisé en ce qu'** au moins une tête d'extrusion se compose d'un dispositif de liquéfaction d'au moins un polymère biothermoplastique couplé à un fil et à une tête de buse.

3. Le système de biofabrication intégré selon la revendication 1, **caractérisé en ce qu'**au moins une tête de dépôt dépose des biomatériaux naturels, des biomolécules/facteurs de croissance et des cellules différenciées et/ou non différenciées.

4. Le système de biofabrication intégré selon la revendication 1, **caractérisé en ce qu'**au moins une tête d'extrusion et au moins une tête de dépôt se déplacent horizontalement et verticalement selon les axes y et z, et la plate-forme de travail coulissante se déplace sur l'axe x.

5. Le système de biofabrication intégré selon la revendication 1, **caractérisé en ce que** la chambre numérique capture une image numérique de la structure tridimensionnelle et détecte avec précision les données pertinentes de celle-ci, qui seront traitées par l'unité de traitement.

6. Le système de biofabrication intégré selon la revendication 1, **caractérisé en ce que** l'unité de traitement effectue l'analyse des données obtenues au moyen de logiciels.

7. Le système de biofabrication intégré selon la revendication 1, **caractérisé en ce que** la seringue et l'aiguille/buse se déplacent horizontalement et verticalement selon les axes y et z, et la plate-forme de travail coulissante se déplace sur l'axe x.

8. Le système de biofabrication intégré selon la revendication 1, **caractérisé en ce que** le bioréacteur effectue des mouvements de déplacement et/ou de rotation.
